Europäisches Patentamt

European Patent Office

Office européen des brevets

(11) Publication number: **0 096 728**
**B1**

(12) **EUROPEAN PATENT SPECIFICATION**

(45) Date of publication of patent specification: **05.03.86**

(21) Application number: **83900066.8**

(22) Date of filing: **23.12.82**

(86) International application number:
**PCT/JP82/00476**

(87) International publication number:
**WO 83/02227 07.07.83 Gazette 83/16**

(51) Int. Cl.⁴: **A 61 K 31/19,** A 61 K 31/195,
A 61 K 31/70

(54) PROPHYLACTIC OR THERAPEUTIC AGENT FOR BLOAT.

(30) Priority: 25.12.81 JP 209198/81
25.12.81 JP 209199/81
25.12.81 JP 209200/81

(43) Date of publication of application:
28.12.83 Bulletin 83/52

(45) Publication of the grant of the patent:
05.03.86 Bulletin 86/10

(84) Designated Contracting States:
FR

(56) References cited:
GB-A- 995 912
US-A-3 010 828
US-A-3 248 289

CHEMICAL ABSTRACTS, vol. 98, no. 13, March
28, 1983, page 497, abstract no. 106007w
COLUMBUS OHIO (US)
CHEMICAL ABSTRACTS, vol. 98, no. 13, March
28, 1983, page 513, abstract no. 106195f
COLUMBUS OHIO (US)

(73) Proprietor: MITSUI TOATSU CHEMICALS,
INCORPORATED
2-5 Kasumigaseki 3-chome
Chiyoda-Ku Tokyo 100 (JP)

(72) Inventor: KAWASHIMA, Ryoji
Shimogamo-matsunoki-cho Sakyo-ku
Kyoto-shi Kyotofu 606 (JP)
Inventor: USAGAWA, Tomoya
HA 31, Aza Higashino Osimizu-cho
Hakui-gun Ishikawaken 929-13 (JP)
Inventor: MASUDA, Takayoshi
4-303, Mukaihazama 1-2 Nawa-cho
Tokai-shi Aichiken 476 (JP)
Inventor: WATANABE, Yoshimoto
3078-78, Matsushin-cho 3-chome
Kasugai-shi Aichiken 486 (JP)

(74) Representative: Kohn, Armand
5 Avenue Foch
F-92380 Garches (FR)

(58) References cited:

**PATENTS ABSTRACTS OF JAPAN, vol. 6, no.
106, June 16, 1982, page 984 C 108**
**JOURNAL OF DAIRY SCIENCE, vol. 57, no. 7,
July 1974 CHAMPAIGN ILL (US) R.T.J. CLARKE
et al.: "Foamy Bloat of Cattle. A review", pages
753-785**
**DEVELOPMENTS IN FOOD CARBOHYDRATE-2,
C.K. LEE Editor 1980, Applied Science
Publishers Ltd. LONDON (GB) J.R. HURFORD:
"Surface active agents derived from some
selected disaccharides", pages 327-350**

# 0 096 728

**Description**

**Field of the art**

The present invention relates to agents for bloat-prevention or -treatment. More particularly, the present invention relates to agents for bloat-prevention or -treatment which comprise a combination of a saccharide fatty acid ester and/or a fatty acid salt.

**Background of the art**

Bloat is a disease that a rumen and a reticulum of ruminants, such as cattle, sheep, etc., distend severely due to a fermentative gas accumulating therein. Bloat is one of the most horrible diseases in feeding beef cattle, dairy cattle, sheep, etc., because ruminants affected with bloat fall into inappetence to result in the reduction in growth rate or milk yield, and to cite an extreme case, they are suffocated to death.

Though there have been various theories in relation to the cause of bloat, the established theory is today that the feeding of much legume or the feeding of much concentrate causes bloat. Namely, it is considered as follows: Much legume being fed, the contents of a rumen become liable to foam owing to the actions of foaming substances, such as saponin, vegetable protein, etc., contained in legume, and a roughage being fed insufficiently and much concentrate being fed, an abnormal fermentation in a rumen, the viscosity increase of a liquid contained therein, etc., become liable to occur, so that in the both cases the exhaustion of gas by eructation is impeded to result in the excessive distensions of a rumen and a reticulum.

There are known methods for preventing or treating bloat, for example, (1) a method in which much oil is sprinkled on a pastureland, (2) a method in which the amount of a roughage used together with a concentrate is kept proper in proportion to the amount of the concentrate, (3) a method in which a drinking water or a mineral block, added with a defoaming agent such as silicone, polypropylene glycol (another name: polyoxypropylene glycol), polyoxypropylene/polyoxyethylene block-copolymer, etc., is always used, (4) a treating method in which a stomach catheter or a trocar is used for exhausting gas, (5) a treating method in which the administration of much defoaming agent such as silicone, polypropylene glycol, polyoxypropylene/polyoxyethylene block-copolymer, mineral oil, vegetable oil, etc., is employed. However, these methods have the following disadvantages respectively: Namely, the method (1) requires much labor and is not economical, because of sprinkling oil on a large pastureland, the method (2) has a problem that this method is inconsistent with a system for fattening beef cattle rapidly by feeding much concentrate, said system being employed in Japan, and it is difficult to obtain excellent results by this method, and the method (4) requires an expert and is attended with a strong possibility of the relapse which is repeated so long as a cause of bloat is not removed. On the other hand, though the methods (3) and (5) can show a considerable good effect, a problem in using most defoaming agents today known is to cause digestion disturbance owing to the long continuous administration of a defoaming agent or owing to the administration of much defoaming agent which is done at a time. Accordingly, it is today expected that an agent with more excellent efficiencies in defoaming effect, etc., and also with an excellent safety, will be produced.

**Disclosure of the invention**

In consideration of such circumstances as described above, the present inventors have continued researches and made the present invention, according to which use is made of a saccharide fatty acid ester, the acid moiety of which has 6 to 24 carbon atoms, or of a salt of such a fatty acid having 6 to 24 carbon atoms, or a combination thereof, for the manufacture of a medicinal agent for the treatment or prevention of bloat. The agent has an excellent defoaming effect, while it lowers the viscosity of rumen juice, raises the pH of the juice, and is very safe for living body.

The present invention is explained in detail below.

The animals intended for the agents for bloat-prevention or -treatment of the present invention are ruminants, such as beef cattle, dairy cattle, young cattle, sheep, goats, etc.

Saccharide fatty acid esters which are used for the present invention, are the fatty acid esters of saccharides, to give typical examples, arabinose, xylose, ribose, lyxose, ribulose, xylulose, glucose, galactose, talose, mannose, fructose, sorbose; tagatose, psicose, maltose, isomaltose, cellobiose, gentiobiose, trehalose, lactose, sucrose, maltotriose, gentianose, raffinose, stachyose, etc. Monoesters, polyesters, such as diesters, triesters, tetraesters, etc., and the mixtures of two or more of them, are usable: monoester has one ester linkage in one molecule of saccharide fatty acid ester; polyester has two or more ester linkages in one molecule of saccharide fatty acid ester.

Among the saccharide fatty acid esters described above, sucrose fatty acid esters are examples of the saccharide fatty acid esters which are most suitable in the present invention, since they are today easily available and also considerably low-priced owing to the industrial mass-productions.

Fatty acids composed of 6—24 carbons comprise the fatty acid moiety of the saccharide fatty acid esters. Both saturated and unsaturated fatty acids are usable. Both fatty acids composed of a straight carbon chain and fatty acids composed of a branched carbon chain are usable. Further, fatty acids with one or more substituents such as a hydroxyl group, are usable. Fatty acids are not necessarily monobasic acids, and polybasic acids such as dibasic acids are also usable.

3

Further, fatty acids are not restricted to fatty acids derived from natural materials such as oils or fats. Synthetic fatty acids are also usable which are produced by the liquid-phase catalytic oxidation of paraffins, the carbonylation of α-olefins (oxo method), the carboxylation of branched olefins (Koch's method) or other methods.

Typical examples of such fatty acids are as follows: caproic acid, enanthic acid, caprylic acid, pelargonic acid, capric acid, lauric acid, tridecanoic acid, 2-methyltetradecanoic acid, 5-methyltetradecanoic acid, 2,2-dimethyltetradecanoic acid, myristic acid, palmitic acid, margaric acid, stearic acid, arachic acid, behenic acid, lignoceric acid, oleic acid, elaidic acid, linoleic acid, linolenic acid, ricinoleic acid, arachidonic acid, eicosapentaenoic acid, erucic acid, azelaic acid, sebacic acid and 1,20-eicosamethylene-dicarboxylic acid.

The saccharide fatty acid esters above described are not necessarily used respectively alone, and mixtures of two or more kinds of saccharide fatty acid esters e.g. wherein the saccharide moieties are different, wherein the fatty acid moieties are different or wherein the numbers of the ester linkages are different, are also usable in any mixing ratio.

On the other hand, the other ingredient to be used for the present invention is the fatty acid salt. Typical examples of fatty acid salts are the salts of various fatty acids described above, enumerated as follows: alkali metal salts such as lithium salts, sodium salts or potassium salts; alkaline earth metal salts such as magnesium salts, calcium salts or barium salts; various metal salts such as zinc salts, aluminum salts, iron salts or manganese salts; ammonium salts; organoamine salts such as monoethanolamine salts, diethanolamine salts or triethanolamine salts; basic amino acid salts such as lysine salts, ornithine salts, arginine salts, histidine salts or hydroxylysine salts most preferably, alkali metal salts, ammonium salts and basic amino acid salts, are used.

As described above in relation to saccharide fatty acid esters, the fatty acid salts above described are respectively usable alone, or alternatively mixtures of two or more kinds of fatty acid salts wherein the fatty acid moieties are different or wherein the cations are different, are also usable.

These saccharide fatty acid esters and fatty acid salts can be easily produced by known methods, and the ones produced by any method are usable in the present invention.

The typical examples for preparing saccharide fatty acid esters are enumerated as follows: (1) saccharide and fatty acid lower-alkyl ester, for example, fatty acid methyl ester, fatty acid ethyl ester, etc., are subjected to alcoholysis in which a fatty acid salt, for example, a fatty acid sodium salt or a fatty acid potassium salt, and a basic catalyst are used in the presence of water or a very safe solvent such as propylene glycol; (2) saccharide and a fatty acid methyl ester, fatty acid carbitol ester or fatty acid glyceride (mono-, di- and triglyceride) are subjected to alcoholysis in the presence of a fatty acid salt, for example, a fatty acid sodium salt, or a fatty acid potassium salt; (3) saccharide and a fatty acid lower-alkyl ester, or oil or fat (that is, fatty acid triglyceride), are subjected to alcoholysis in the presence of a basic catalyst; (4) saccharide is reacted with a fatty acid chloride or a fatty acid anhydride.

Among these examples especially the examples (1),(2) and (3) are very advantageous, because the crude products prepared by these examples contain saccharide fatty acid ester and fatty acid salt (usually, fatty acid alkali metal salt), can be economically produced and are usable in the present invention without purification.

On the other hand, fatty acid salts can be easily produced by reacting a fatty acid or a fatty acid ester, for example, a fatty acid methyl ester, a fatty acid ethyl ester or a fatty acid glyceride, with an oxide, hydroxide, carbonate or hydrogen carbonate of an alkali metal or alkaline earth metal, a basic amino acid, ammonia or organoamine.

The fatty acid salts produced directly by the above-described methods are not necessarily used. For example, it is possible that the fatty acid and oxide, hydroxide, carbonate or hydrogen carbonate of alkali metal, or basic amino acid, are used each in the free state so that the fatty acid salt will be generated on preparation of the agents for bloat-prevention or -treatment of the present invention. Employing such methods is within the scope of the present invention.

The agent may comprise saccharide fatty acid ester alone, or fatty acid salt alone. If the agent comprises a combination of these two ingredients, the ratio by weight of saccharide fatty acid ester to fatty acid salt is usually in the range of 97:3 to 3:97, preferably 95:5 to 5:95, most preferably 90:10 to 10:90.

In the present invention, it is most necessarily required that the saccharide fatty acid esters and the fatty acid salts are purified to a high degree. They may contain one or more substances highly safe for the living body, such as saccharide, fatty acid lower-alkyl ester, fatty acid glyceride (mono-, di- and triglyceride), fatty acid, alkali metal carbonate or basic amino acid, which remain owing to partial unreaction or which may remain from the production of the saccharide fatty acid esters or the fatty acid salts as in the case of the crude saccharide fatty acid ester described above, and byproducts, such as alcohol or glycerin.

The agents for bloat-prevention or -treatment of the present invention can be used in any forms such as powder, granule, pellet, crumble, cube, tablet, half-wetted, paste, aqueous solution or aqueous suspension.

The various forms above described being prepared, the following materials may be used for example as a diluting agent: water; wheat flour, starch, dextrin; widely used materials for feed, for example, cereal grains such as corn and milo (kaoliang); chaffs and brans such as rice bran, defatted rice bran and wheat

4

bran; oil meals such as soybean meal, rapeseed meal, cottonseed meal and linseed meal; oils or fats such as beef tallow, soybean oil, palm oil, coconut oil and fish oil.

When the agents for bloat-prevention or -treatment of the present invention are administered to animals, each agent in a form described above may be administered apart from drinking water and feed, or may be forcibly injected into a rumen.

The addition into drinking water or feed to be given to animals, is generally most convenient. In a feeding system in which pasture is principally used, the agents may be sprinkled on pasture.

Though the dose of the agents for bloat-prevention or -treatment of the present invention can not be uniformly fixed, because of variation of the factors such as, kind, age and body weight of animals, the intended use of the present agents, namely, using as an agent for prevention or using as an agent for treatment, administration method, the extent of bloat, kind of feeds, the suitable total dose of agent is usually, for example, when being added into drinking water or feed to be given to animals, approximately 0.005—10% by weight, preferably 0.01—5% by weight, most preferably 0.02—3% by weight to the amount of drinking water or feed which is finally given to animals. In the case that the dose of agent is less than the lowest limit value described above, it becomes difficult to show sufficiently the effect of the present invention, and the dose exceeding the highest limit value described above do not have any specific effect and are rather uneconomical, so that such uses are not desirable.

When the present agents are used for prevention, a sufficient effect can be produced by a continuous administration even if in a relatively low concentration. When the present agents are used for treatment, the effect of the present invention can be produced in a short period by employing a higher concentration than in using for prevention.

The agents for bloat-prevention or -treatment of the present invention are usually used alone and can be naturally used together with a known defoaming agent such as, silicone, polypropylene glycol, polyoxypropylene/polyoxyethylene block-copolymer, oil or fat, or other agent.

Having a much more excellent defoaming effect than conventional agents, the agents for bloat-prevention or -treatment of the present invention can prevent foaming of rumen juice of ruminants and can restore a foamed rumen juice to a normal state. Saccharide fatty acid esters and fatty acid salts both have good defoaming effect. The use of a combination both of these can produce an even better excellent defoaming effect owing to synergism. The defoaming effect can be further heightened by using a saccharide fatty acid ester and a fatty acid salt, together with a fatty acid glyceride, for example, oil or fat (that is, fatty acid triglyceride), a fatty acid monoglyceride, fatty acid diglyceride, or a propylene glycol fatty acid ester.

The agents for bloat-prevention or -treatment of the present invention have the effect of exhausting immediately gas produced in the rumen through the mouth out of the body, because the agents have the effect of lowering an established ingesta volume expansion rate and the viscosity of rumen juice. This established ingesta volume expansion rate represents the relative easiness of the exhaustion of a fermentative gas produced in a rumen (lowering of said value means that the exhaustion of the gas becomes easy).

Further, the agents for bloat-prevention or -treatment of the present invention have an action of raising the pH value of rumen juice to a desirable level. The pH value of the rumen juice of healthy ruminants is usually about 6.5—7.5. However, much concentrate having been fed and bloat having been induced e.g. by an abnormal fermentation in a rumen, it is frequently observed that the pH value is liable to be lowered (there are various types of bloats, so that certain bloats show almost the same pH value as in a healthy state) and it is not rare that the pH value lowers down to about 4—5. Further, the extreme lowering of a pH value of rumen juice is a serious problem, because such an extreme lowering of pH value is apt to kill microorganisms in a rumen, such as bacteria and protozoa.

The agents of the present invention have the effect of raising the pH value of rumen juice to a desirable level. This action keeps or recovers an environment suitable for microorganisms in a rumen and has the effect for preventing or treating bloat together with the above described defoaming action.

Furthermore, the agents for bloat-prevention or -treatment of the present invention are very safe for the living body and are also easily metabolized in the living body. Therefore, a long continuous administration to animals and the administration of a large dose, do not become a problem from the point of view of safety.

As described above, the agents for bloat-prevention or -treatment of the present invention have various properties, such as an excellent defoaming effect, an effect lowering viscosity of rumen juice of ruminants, an effect raising the pH value to a desirable degree, and have an excellent safety, and therefore have a high utilizability as an agent for bloat-prevention or -treatment.

Hereinafter, the present invention is in detail explained by means of examples, controls and references.

Examples 1—6

In order to examine mimetically the administration effects, especially the defoaming effects, of the agents for bloat-prevention or -treatment of the present invention against bloat which is frequently induced by the feeding of much legume, the various agents of the present invention comprising saccharide fatty acid ester, shown in Table 1, were severally added into each aqueous saponin solution so as to become each specified concentration (shown in Table 1) to each aqueous saponin solution of 0.25% by weight, and

5

the foamabilities of the resulted solutions were measured. The results were shown in Table 1. The method for measuring the foamability is shown as follows:

Method for measuring the foamability

The agent for bloat-prevention or -treatment of the present invention is added into an aqueous saponin solution of 0.25% by weight in a specified amount and mixed. Just and five minutes after foaming treatment at 25°C or 40°C according to Ross & Miles's method (Japanese Industrial Standard JIS K 3362), each resulted foam height (mm) is measured.

Controls 1 and 2

The foamabilities of only the aqueous saponin solution of 0.25% by weight without any agent for bloat-prevention or -treatment of the present invention, were measured in each control in the same manner as used in examples 1—6 (measured temperature; 25°C in control 1; 40°C in control 2). The results were shown in Table 1.

TABLE 1

The composition and the use amount of the agents for bloat-prevention or -treatment of the present invention

| Example or Control | Kind of saccharide fatty acid esters | Conc. (% by weight) | Measurement temperature (°C) | Foamability (mm) | |
|---|---|---|---|---|---|
| | | | | Just after | Five minutes after |
| Example 1 | Sucrose hydrogenated beef tallow fatty acid ester mono-: 70% by weight) di-: 30% by weight | 0.25 | 25 | 35 | 29 |
| Example 2 | " | 0.05 | 25 | 56 | 45 |
| Example 3 | " | 0.05 | 40 | 32 | 25 |
| Example 4 | Sucrose palm oil fatty acid ester mono-: 50% by weight di-: 30% by weight tri-: 20% by weight | 0.05 | 40 | 30 | 24 |
| Example 5 | Raffinose beef tallow fatty acid ester mono-: 60% by weight di-: 30% by weight tri-: 10% by weight | 0.25 | 25 | 31 | 22 |
| Example 6 | Maltotriose mono-palmitic acid ester | 0.25 | 25 | 30 | 22 |
| Control 1 | | | 25 | 220 | 195 |
| Control 2 | | | 40 | 228 | 204 |

Reference 1

A commercially available agent for bloat-prevention (polyoxypropylene/polyoxyethylene block-copolymer, molecular weight: 1250, the content of polyoxyethylene parts: 20%) was added so as to become 0.25% by weight in the aqueous saponin solution of 0.25% by weight. The foamabilities of the resulted solution were measured in the same manner as in Examples 1—6 (measured temperature: 25°C). The foamability was 65 mm (just after foaming treatment) and 57 mm (five minutes after foaming treatment).

Reference 2

The same commercially available agent for bloat-prevention as used in reference 1, was added so as to become 0.05% by weight in the aqueous saponin solution of 0.25% by weight. The foamabilities of the resulted solution were measured in the same manner as in Examples 1—6 (measured temperature: 25°C). The foamability was 172 mm (just after foaming treatment) and 152 mm (five minutes after foaming treatment).

Example 7

Ten Holstein bullocks weighing about 450 kg were divided into two groups (test group and control group) respectively consisting of five Holstein bullocks, and feeding trials were carried out by feeding (free feeding) the respective concentrates with each composition shown in Table 2 to the groups for four weeks.

As the result, in the control group the abdomens of two bullocks distended extremely intermittingly and a slight bloat appeared, but in the test group all bullocks were not affected with bloat.

Further, at the second week and at the fourth week in the testing period, rumen juices were collected from all bullocks to measure the viscosities. The average viscosities were 9.4 cp/25°C in the test group and 15.1 cp/25°C in the control group (each is an average of the measured values at the second week and at the fourth week).

TABLE 2

Compositions of given concentrates (parts by weight)

| | Basic feed (Note 1) | Sucrose hydrogenated beef tallow fatty acid ester (monoester: 70% by weight diester: 30% by weight) |
|---|---|---|
| Test group | 100 . | 1 |
| Control group | 100 | 0 |

Note 1. DCP (digestible crude protein): 9.3%
TDN (total digestible nutrient): 76%

Examples 8—13

In order to examine mimetically the administration effects, especially the defoaming effects, of the agents for bloat-prevention or -treatment of the present invention against bloat which is frequently induced by the feeding of much legume, the various agents of the present invention comprising fatty acid salt, shown in Table 3, were severally added into each aqueous saponin solution so as to become each specified concentration (shown in Table 3) to each aqueous saponin solution of 0.25% by weight, and the foamabilities of the resulted solutions were measured. The results were shown in Table 3. The method for measuring the foamability is shown as follows:

Method for measuring the foamability

The same method as shown in Examples 1—6 was used.

Controls 3 and 4

Controls 3 and 4 were carried out in the same manner as in controls 1 and 2, provided that the measured temperature was 25°C in control 3 and 40°C in control 4. The results were shown in Table 3.

Reference 3

A commercially available agent for bloat-prevention (polyoxypropylene/polyoxyethylene block-copolymer, molecular weight: 1250, the content of polyoxyethylene parts: 20%) was added so as to become 0.25% by weight in the aqueous saponin solution of 0.25% by weight. The foamabilities of the resulted solution were measured in the same manner as in Examples 8—13 (measured temperature: 25°C). The foamability was 65 mm (just after foaming treatment) and 57 mm (five minutes after foaming treatment).

7

TABLE 3
The composition and the use amount of the agents for bloat-prevention or
-treatment of the present invention

| Example or Control | Kind of fatty acid salts | Conc. (% by weight) | Measurement temperature (°C) | Foamability (mm) | |
| --- | --- | --- | --- | --- | --- |
| | | | | Just after | Five minutes after |
| Example 8 | Stearic acid potassium salt | 0.25 | 25 | 40 | 31 |
| Example 9 | " | 0.25 | 40 | 35 | 27 |
| Example 10 | Beef tallow fatty acid sodium salt | 0.25 | 40 | 41 | 30 |
| Example 11 | " | 0.10 | 40 | 58 | 40 |
| Example 12 | Beef tallow L-lysin salt | 0.25 | 40 | 47 | 35 |
| Example 13 | Palm oil fatty acid L-arginine salt | 0.25 | 40 | 42 | 30 |
| Control 3 | | | 25 | 220 | 195 |
| Control 4 | | | 40 | 228 | 204 |

Example 14

Ten Holstein bullocks weighing about 450 kg were divided into two groups (test group and control group) respectively consisting of five Holstein bullocks, and feeding trials were carried out by feeding (free feeding) the respective concentrates with each composition shown in Table 4 to the groups for four weeks.

As the result, in the control group the abdomens of two bullocks distended extremely intermittently and a slight bloat appeared, but in the test group all bullocks were not affected with bloat.

Further, at the second week and at the fourth week in the testing period, rumen juices were collected from all bullocks to measure the pH values. The average viscosities were 7.2 in the test group and 6.5 in the control group (each is an average value of the measured values at the second week and at the fourth week).

TABLE 4
Compositions of given concentrates (parts by weight)

| | Basic feed (Note 1) | Beef tallow fatty acid potassium salt |
| --- | --- | --- |
| Test group | 100 | 2 |
| Control group | 100 | 0 |

Note 1. DCP (digestible crude protein): 9.3%
TDN (total digestible nutrient): 76%

Examples 15—28

In order to examine mimetically the administration effects, especially the defoaming effects, of the agents for bloat-prevention or -treatment of the present invention against bloat which is frequently induced by the feeding of much legume, the various agents of the present invention comprising saccharide fatty acid ester and fatty acid salt, shown in Table 5, were saverally and fatty acid salt, shown in Table 5, were severally added into each aqueous saponin solution so as to become each specified concentration (shown in Table 5) to each aqueous saponin solution of 0.25% by weight, and the foamabilities of the resulted solutions were measured (proviso: in each case of Examples 25—28, the crude product prepared by the reaction, which contains fatty acid glyceride, sucrose, glycerin, etc., besides saccharide fatty acid ester and fatty acid salt, was added, and then the foamabilities were measured). The results were shown in Table 5. The method for measuring the foamability is shown as follows:

Method for measuring the foamability
The same method as shown in Examples 1—6 was used.

8

Controls 5 and 6

Controls 5 and 6 were carried out in the same manner as in controls 1 and 2, provided that the measured temperature was 25°C in control 5 and 40°C in control 6. The results were shown in Table 5.

Control 7

Only saccharide fatty acid ester was added into an aqueous saponin solution of 0.25% by weight and the foamabilities of the resulted solution were measured in the same manner as in Examples 15—28 (measured temperature: 25°C). The results were shown in Table 5.

Control 8

Only fatty acid salt was added into an aqueous saponin solution of 0.25% by weight and the foamabilities of the resulted solution were measured in the same manner as in Examples 15—28 (measured temperature: 25°C). The results were shown in Table 5.

Reference 4

A commercially available agent for bloat-prevention (polyoxypropylene/polyoxyethylene block-copolymer, molecular weight: 1250, the content of polyoxyethylene parts: 20%) was added so as to become 0.25% by weight in the aqueous saponin solution of 0.25% by weight. The foamabilities of the resulted solution were measured in the same manner as in Examples 15—28 (measured temperature: 25°C). The foamability was 65 mm (just after foaming treatment) and 57 mm (five minutes after foaming treatment).

Reference 5

The same commercially available agent for bloat-prevention as used in reference 4, was added so as to become 0.05% by weight in the aqueous saponin solution of 0.25% by weight. The foamabilities of the resulted solution were measured in the same manner as in Examples 15—28 (measured temperature: 25°C). The foamability was 172 mm (just after foaming treatment) and 152 mm (five minutes after foaming treatment).

TABLE 5
The composition and the use amount of the agents for bloat-prevention or
-treatment of the present invention

| Example or Control | Saccharide fatty acid ester (Note 1) | | Fatty acid salt (Note 2) | | Measurement temperature (°C) | Foamability (mm) | |
|---|---|---|---|---|---|---|---|
| | Kind | Conc. (% by weight) | Kind | Conc. (% by weight) | | Just after | Five minutes after |
| Example 15 | Sucrose hydrogenated beef tallow fatty acid ester (mono-: 70% by w. di-: 30% by w. ) | 0.20 | Stearic acid potassium salt | 0.05 | 25 | 23 | 20 |
| Example 16 | " | 0.20 | " | 0.05 | 40 | 22 | 19 |
| Example 17 | " | 0.05 | " | 0.20 | 25 | 23 | 21 |
| Example 18 | " | 0.05 | " | 0.20 | 40 | 24 | 22 |
| Example 19 | " | 0.033 | " | 0.017 | 25 | 21 | 18 |
| Example 20 | " | 0.017 | " | 0.033 | 25 | 23 | 20 |
| Example 21 | " | 0.20 | Coconut fatty acid sodium salt | 0.05 | 25 | 16 | 12 |

0 096 728

TABLE 5 (Cont'd)

| Example or Control | Saccharide fatty acid ester (Note 1) | | Fatty acid salt (Note 2) | | Measurement temperature (°C) | Foamability (mm) | |
|---|---|---|---|---|---|---|---|
| | Kind | Conc. (% by weight) | Kind | Conc. (% by weight) | | Just after | Five minutes after |
| Example 22 | Sucrose beef tallow fatty acid ester mono-: 65% by w. $\left(\begin{array}{l}\text{di-: 30\% by w.}\\\text{tri-: 5\% by w.}\end{array}\right)$ | 0.05 | Palmitic acid L-lysin salt | 0.05 | 25 | 25 | 20 |
| Example 23 | Raffinose beef tallow fatty acid ester mono-: 60% by w. $\left(\begin{array}{l}\text{di-: 30\% by w.}\\\text{tri-: 10\% by w.}\end{array}\right)$ | 0.15 | Oleic acid sodium salt | 0.10 | 40 | 14 | 9 |
| Example 24 | Maltotriose monopalmitic acid ester | 0.10 | Beef tallow fatty acid sodium salt | 0.05 | 40 | 20 | 15 |
| Example 25 | The crude product (Note 3) prepared by subjecting sucrose and beef tallow to alcoholysis in which potassium carbonate is used as a catalyst, was added so as to become 0.25% by weight. | | | | 25 | 0 | 0 |
| Example 26 | " | | | | 40 | 0 | 0 |
| Example 27 | The same crude product as used in Examples 24 and 25, was added so as to become 0.05% by weight. | | | | 25 | 11 | 0 |

TABLE 5 (Cont'd)

| Example or Control | Saccharide fatty acid ester (Note 1) | | Fatty acid salt (Note 2) | | Measurement temperature (°C) | Foamability (mm) | |
|---|---|---|---|---|---|---|---|
| | Kind | Conc. (% by weight) | Kind | Conc. (% by weight) | | Just after | Five minutes after |
| Example 28 | The same crude product as in Examples 24 and 25, was added so as to become 0.05% by weight. | | | | 40 | 6 | 0 |
| Control 5 | | | | | 25 | 220 | 195 |
| Control 6 | | | | | 40 | 228 | 204 |
| Control 7 | Sucrose hydrogenated beef tallow fatty acid ester mono-: 70% by w. di-: 30% by w. | 0.05 | | | 25 | 56 | 45 |
| Control 8 | | | Stearic acid potassium salt | 0.05 | 25 | 212 | 183 |

Notes 1 and 2. All the concentrations mean each concentration of saccharide fatty acid esters or fatty acid salts in an aqueous saponin solution of 0.25% by weight (proviso: the concentration of the crude product prepared by the reaction was shown in each of Examples 25—28).

Note 3. The crude product prepared by the reaction which comprises sucrose beef tallow fatty acid ester 30% by weight (monoester/diester=65/35 weight ratio), beef tallow fatty acid potassium salt 25% by weight, beef tallow fatty acid glyceride 20% by weight (the total amount of mono-, di- and triglyceride) and others 25% by weight (sucrose, glycerin, etc.), was used in Examples 25—28.

Reference 6

A commercially available agent for bloat-prevention (polyoxypropylene/polyoxyethylene block-copolymer, molecular weight: 2000, the content of polyoxyethylene parts: 50%) was added so as to become 0.25% by weight in the aqueous saponin solution of 0.25% by weight. The foamabilities of the resulted solution were measured in the same manner as in Examples 15—28 (measured temperature: 25°C). The foamability was 185 mm (just after foaming treatment) and 168 mm (five minutes after foaming treatment).

Examples 29—31

It is shown by the following experiments that a fermentative gas is easily exhausted by adding the agents for bloat-prevention or -treatment of the present invention into rumen juice of a ruminant.

Experiment method

Rumen juices are collected from three sheep affected with bloat artificially by feeding a bloat-producing feed (described in "D. R. Jacobson et al., Journal of Animal Science, Vol. 16, pages 515—524 (1957)", comprising 61% barley, 22% alfalfa meal, 16% soybean meal and 1% NaCl). These rumen juices are mixed together and used as the rumen juice in this experiments.

Then, every 200 ml of this mixture is poured into 500 ml measuring cylinders and thereinto the agent for bloat-prevention or -treatment of the present invention is added in each specified amount. Thereafter each resulted mixture is incubated at 39°C for one hour in an incubator and further incubated with stirring by a glass rod every five minutes for one hour and the volume increase ratio (%) of the rumen juice in the measuring cylinder is measured, and the value of this ratio is used as the sable ingesta volume increase (referred to as Stable IVI hereinafter). Stable IVI teaches that the smaller Stable IVI becomes, the easier exhausting a fermentative gas out of rumen juice becomes.

Experiment conditions and results

The experiment conditions and results were shown in Table 6. In the present experiments, the agent for bloat-prevention or -treatment of the present invention is the crude product prepared by subjecting sucrose and beef tallow to alcoholysis in which potassium carbonate is used as a catalyst and said crude product is the same as used in Examples 25—28.

Control 9

Stable IVI of only the rumen juice without the agent for bloat-prevention or -treatment of the present invention was measured in the same manner as in Examples 29—31. The result was shown in Table 6.

Example 32

Eighteen Holstein bulls of eight weeks old were divided into three groups respectively consisting of six Holstein bulls, and feeding trials were carried out by feeding the respective feeds with each composition shown in Table 7 to the groups for five weeks (namely, till thirteen weeks old). Besides the respective feeds with each composition shown in Table 7, hay was fed as a roughage to the bulls of all groups at the rate of about 0.3 kg/day/bull (this amount corresponds to about one-tenth each feeding amount of the feeds shown in Table 7). During the test period, the rumen juices of the bulls of all groups were collected at 10 and 13 weeks old, and the viscosities and the pH values were measured. The averages of viscosities and pH values of these rumen juices in each group were calculated and shown in Table 7.

TABLE 6

Agent for bloat-prevention or -treatment of the present invention

| Example or Control | Kind | Concentration to the rumen juice (% by weight) | Stable IVI (%) |
|---|---|---|---|
| Example 29 | The same crude product as used in Examples 25—28 | 0.5 | 21 |
| Example 30 | '' | 1 | 19 |
| Example 31 | '' | 2 | 20 |
| Control 9 | | | 47 |

## 0 096 728

### TABLE 7

| Group Division | 1 Test No. 1 | 2 Test No. 2 | 3 Control |
|---|---|---|---|
| **Ingredients and mixing ratio (parts by weight) in each given feed** | | | |
| Commercially available feed for young cattle (Note 1) | 100 | 100 | 100 |
| Agent for bloat-prevention or -treatment of the present invention (Note 2) | 1 | 0.2 | 0 |
| **State of each rumen juice** | | | |
| Viscosity (cp/25°C) | 7.7 | 9.5 | 13.5 |
| pH | 7.1 | 6.7 | 6.5 |

Note 1. DCP (digestible crude protein): 18%
TDN (total digestible nutrient): 68%
Note 2. The same crude product as used in Examples 25—31, was used.

### Example 33

Ten Holstein bullocks weighing about 450 kg were divided into two groups (test group and control group) respectively consisting of five Holstein bullocks, and feeding trials were carried out by feeding (free feeding) the respective concentrates with each composition shown in Table 8 to the groups for four weeks. As the result, in the control group the abdomens of two bullocks distended extremely intermittingly and a slight bloat appeared, but in the test group all bullocks were not affected with bloat.

### TABLE 8
Compositions of given concentrates (parts by weight)

| | Basic feed (Note 1) | Agent for bloat-prevention or -treatment of the present invention (Note 2) |
|---|---|---|
| Test group | 100 | 0.5 |
| Control group | 100 | 0 |

Note 1. DCP (digestible crude protein): 9.3%
TDN (total digestible nutrient): 76%
Note 2. The same crude product as used in Examples 25—32, was used.

### Example 34

Twenty five g of the same crude product as used in Examples 25—33 was diluted tenfold with water, the obtained crude product solution was compulsorily administered perorally into one bullock with a chronic serious bloat (Holstein bullock weighing about 500 kg, fed by a system in which much concentrate is fed), and then the change of his girth with the passage of time was measured. The results in Table 9 shown below were obtained. One hour after the administration, the distension of his abdomen vanished thoroughly and he was restored to health.

### TABLE 9

| Time after the administration (hour) | Girth (cm) |
|---|---|
| 0 | 260 |
| 0.5 | 241 |
| 1.0 | 230 |

14

Example 35

Fifty g of the same crude product as used in Examples 25—34, was diluted tenfold with water, the obtained crude product solution was compulsorily administered perorally into one bullock with a chronic serious bloat (Japanese black-haired bullock, weight: about 500 kg), and then the change of his girth with the passage of time, was measured. The results in Table 10 shown below were obtained, and the effect of treating bloat was shown clearly.

TABLE 10

| Time after the administration (hour) | Girth (cm) |
|---|---|
| 0 | 235 |
| 1.0 | 217 |

Example 36

One Japanese brown-haired bullock (weight: about 650 kg) equipped with a fistula was fed with 8 kg/day of a feed (1) (the same bloat-producing feed as used in Examples 29—31, comprising 61% barley, 22% alfalfa meal, 16% soybean meal and 1% NaCl) for two weeks and thereafter consecutively fed with 8 kg/day of a feed (2) (the feed that the same crude product as used in Examples 25—35 was added into the feed (1) in an amount of 1%) for two weeks as in the case of the feed (1). The rumen juices were collected 4, 8, 11 and 14 days after the start of each feeding and the viscosities and Stable IVls of them were measured. The other observations were also carried out. The results were shown in Table 11. These results showed that the agent of the present invention has a remarkable effect on bloat-prevention.

TABLE 11

| | | State of the rumen juice (*) | | | |
|---|---|---|---|---|---|
| | | Viscosity (cp) | | | |
| Feed given | Feeding period | Before filtration (**) | After filtration (***) | Stable IVI (%) | Other observation |
| Feed (1) (only the bloat-producing feed) | 2 weeks | 12.1 | 6.2 | 6.0 | The fistula was separated three times owing to the rise in the internal pressure of the rumen, during the test period. |
| Feed (2) (the bloat-producing feed +1% the agent of the present invention) | " | 7.8 | 3.9 | 0 | Without any trouble. |

Note:

* The average value of four times (4, 8, 11 and 14 days after)
** The measurements were carried out before filtration with double gauzes.
*** The measurements were carried out after the elimination of admixtures with double gauzes.

**Claims**

1. The use of a saccharide fatty acid ester, the acid moiety of which has 6 to 24 carbon atoms, or of a salt of such a fatty acid having 6 to 24 carbon atoms, or a combination thereof, for the manufacture of a medicinal agent for the treatment or prevention of bloat.

2. Use according to claim 1, characterized in that the saccharide is sucrose, raffinose or maltotriose.

3. Use according to claim 1 or 2, characterized in that the salt is a salt of alkali metal or of a basic amino acid.

4. Use according to any of claims 1 to 3, characterized in that it comprises a crude material formed by subjecting a saccharide and a lower alkyl fatty acid ester or fatty acid triglyceride to alcoholysis in the presence of a basic catalyst.

**0 096 728**

5. Use according to claim 4, characterized in that the saccharide is sucrose and the fatty acid triglyceride is an oil or a fat, in particular beef tallow.

**Patentansprüche**

1. Die Verwendung eines Saccharid- Fettsäureesters, dessen Säurerest 6 bis 24 Kohlenstoffatome hat, oder eines Salzes einer solchen Fettsäure mit 6 bis 24 Kohlenstoffatomen, oder einer Kombination davon, zur Herstellung eines Heilmittels zur Behandlung oder Vorbeuge des Aufblähens.

2. Verwendung nach Anspruch 1, dadurch gekennzeichnet, daß das Saccharid Saccharose, Raffinose oder Maltotriose ist.

3. Verwendung nach Anspruch 1 oder 2, dadurch gekennzeichnet, daß das Salz ein Salz eines Alkalimetalls oder einer basischen Aminosäure ist.

4. Verwendung nach einem der Ansprüche 1 bis 3, dadurch gekennzeichnet, daß sie ein Rohmaterial umfaßt, gebildet durch Unterwerfen eines Saccharids und eines niedrigen Alkylfettsäureesters oder Fettsäuretriglyzerids der Alkoholyse, in Anwesenheit eines basischen Katalysators.

5. Verwendung nach Anspruch 4, dadurch gekennzeichnet, daß das Saccharid Saccharose ist und das Fettsäure- triglyzerid ein Öl oder Fett, insbesondere Rindertalg ist.

**Revendications**

1. Utilisation d'un ester d'acide gras de saccharide, dont le radical acide comporte de 6 à 24 atomes de carbone, ou d'un sel de cet acide gras ayant 6 à 24 atomes de carbone, ou bien d'une combinaison de ces produits, pour la fabrication d'un agent médicamenteux pour le traitement ou la prévention de la tympanite.

2. Utilisation selon la revendication 1, caractérisée en ce que le saccharide est sucrose, raffinose ou maltotriose.

3. Utilisation selon la revendication 1 ou 2, caractérisée en ce que le sel est un sel de métal alcalin ou d'un aminoacide basique.

4. Utilisation selon une des revendications 1 à 3, caractérisée en ce qu'il s'agit d'une substance brute formée en soumettant un saccharide et un ester d'acide gras d'un alkyle inférieur, ou un triglycéride d'acide gras, à une alcoolyse en présence d'un catalyseur basique.

5. Utilisation selon la revendication 4, caractérisée en ce que le saccharide est du sucrose et le triglycéride d'acide gras est une huile ou une graisse, en particulier du suif de boeuf.